(19) Europäisches Patentamt

European Patent Office

Office européen des brevets

(11) Veröffentlichungsnummer: **0 518 155 A1**

# (12) EUROPÄISCHE PATENTANMELDUNG

(21) Anmeldenummer: **92109195.5**

(22) Anmeldetag: **01.06.92**

(51) Int. Cl.5: **C07D 277/72**

(30) Priorität: **11.06.91 DE 4119184**
**15.08.91 DE 4126992**

(43) Veröffentlichungstag der Anmeldung:
**16.12.92 Patentblatt 92/51**

(84) Benannte Vertragsstaaten:
**BE DE FR GB IT**

(71) Anmelder: **BAYER AG**

**W-5090 Leverkusen 1 Bayerwerk(DE)**

(72) Erfinder: **Reinartz, Klaus, Dr.**
**Forststrasse 60**
**W-5000 Köln 71(DE)**
Erfinder: **Schlesmann, Harro, Dr.**
**Eifgenstrasse 24**
**W-5068 Odenthal(DE)**
Erfinder: **Dietl, Stefan, Dr.**
**Bitterstrasse 6**
**W-5000 Köln 71(DE)**

(54) **Verfahren zur Reinigung von rohem 2-Mercaptobenzthiazol.**

(57) Rohes 2-Mercaptobenzthiazol wird gereinigt, indem man das rohe 2-Mercaptobenzthiazol in höhersiedenden aliphatischen oder cycloaliphatischen Alkoholen oder in Mischungen aus den genannten Alkoholen oder deren Azeotropen mit Wasser bei Temperaturen von 60 bis 200°C gegebenenfalls unter erhöhtem Druck löst oder in höhersiedenden aliphatischen oder cycloaliphatischen Alkoholen, aliphatischen oder cycloaliphatischen Ethern, organischen Säuren oder deren Estern bei Temperaturen von 10 bis 100°C aufschlämmt, anschließend die Lösung oder die Aufschlämmung abkühlt und das ausfallende Produkt abfiltriert und gegebenenfalls wäscht.

EP 0 518 155 A1

Die vorliegende Erfindung betrifft ein Verfahren zur Reinigung von rohem 2-Mercaptobenzthiazol. Nach dem Verfahren der vorliegenden Erfindung erhält man das 2-Mercaptobenzthiazol in hoher Reinheit und in guter Ausbeute.

Verfahren zur Reinigung der Rohschmelze des 2-Mercaptobenzthiazols sind in der Literatur bereits mehrfach beschrieben worden. Das bislang am häufigsten angewendete Verfahren ist das Auflösen der heißen Rohschmelze in einer heißen alkalischen Lösung und das anschließende Fällen durch Zugabe einer Säure (US 1 631 871, US 2 137 820, US 2 161 741, DD 233 127). Die zusätzliche Reinigung dieser Lösung durch Einleiten von Luft, auch in Gegenwart von Aktivkohle (GB 1 321 181), ist ebenfalls beschrieben worden (US 2 631 153). In einem ähnlichen Verfahren (DOS 2 258 484) wird das rohe 2-Mercaptobenzthiazol zuerst aus einem aromatischen Kohlenwasserstoff umkristallisiert und anschließend in wäßriger Natronlauge aufgelöst. In einem weiteren Verfahren wird die heiße Rohschmelze zuerst in einem inerten Lösungsmittel gelöst und das 2-Mercaptobenzthiazol aus dieser Lösung mit wäßriger Alkalihydroxidlösung extrahiert und durch Ansäuern ausgefällt (DOS 1 941 379). Die allen Verfahren gemeinsamen Nachteile sind der Anfall eines stark belasteten Abwassers und der hohe Bedarf an Alkali und Säure, da diese nicht in den Prozeß zurückgeführt werden können.

Desweiteren sind in der Literatur Verfahren zur Umkristallisation des rohen 2-Mercaptobenzthiazols beschrieben worden. Dabei werden Lösungsmittel z.B. Anilin (EP 169 107, US 4 647 669), Chlor- oder Nitrobenzol (SU 852 869), Kohlenwasserstoffe im Gemisch mit 5 % Phenol und 1 % Pyridin (DE 541 295), ein Gemisch aus Aceton, Wasser und Schwefelsäure (SU 476 266) oder verdünntes Ethanol bzw. 50 %ige Essigsäure (J. prakt. Chem. 93 - (1916) 183) eingesetzt. Diese Verfahren sollen zwar alle zu guten bis sehr guten Ergebnissen führen, haben jedoch entweder den Nachteil, daß strenge Sicherheitsvorschriften einzuhalten sind, oder aber, daß die Aufarbeitung der Mutterlauge sehr kostenintensiv ist. Dies gilt auch für das Reinigungsverfahren, das in den Patenten US 3 030 373 und Ca 448 209 beschrieben wird, wonach 2-Mercaptobenzthiazol in einem Gemisch aus Wasser und einem wasserunlöslichen Lösungsmittel, wie Schwefelkohlenstoff, Toluol oder Benzol, bei erhöhter Temperatur und erhöhtem Druck suspendiert wird. Auch die Wasserdampfdestillation (DE 936 039, US 2 658 864, GB 734 970) zur Abtrennung von Verunreinigungen, wie z.B. Benzthiazol, erscheint in diesem Zusammenhang als ein relativ aufwendiges Verfahren.

Die Flüssig-Flüssig-Extraktion (DOS 2 652 394) erscheint ebenfalls ein recht kompliziertes Verfahren zu sein, da das Einleiten der flüssigen und damit sehr heißen Rohschmelze in kalten Schwefelkohlenstoff besondere Sicherheitsmaßnahmen erfordert.

Die in DOS 2 022 569 beschriebene vorgeschaltete Verdampfung ist für eine großtechnische Auslegung wohl auch nicht sonderlich gut geeignet, zumal anschließend eine weitere Reinigung des kondensierten Produkts nötig ist, die wiederum über Auflösen in wäßriger Natronlauge, Extraktion der Lösung mit einem nicht wasserlöslichen Lösungsmittel und Fällen des 2-Mercaptobenzthiazols durch Ansäuern der wäßrigen Phase erfolgt.

Das Aufschlämmen des rohen 2-Mercaptobenzthiazols in Toluol - evtl. unter Zugabe von Pyridin, Picolin oder Anilin - bei 30°C (JP 6025979) ergibt - auch nach zweimaliger Wiederholung - nur ein Produkt minderer Qualität. Auch die Extraktion des rohen 2-Mercaptobenzthiazols mit Tetrachlorkohlenstoff oder Tetrachlorethylen ist beschrieben worden (JP 7919976). Desweiteren wurde ein Verfahren beschrieben (JP 6025980), die Rohschmelze durch langsame Kristallisation der Schmelze zu reinigen. Dabei sollen die Verunreinigungen in der sich ergebenden Mutterlauge bleiben.

Es wurde nun ein Verfahren zur Reinigung von rohem 2-Mercaptobenzthiazol gefunden, das dadurch gekennzeichnet ist, daß man das rohe 2-Mercaptobenzthiazol in höhersiedenden aliphatischen oder cycloaliphatischen Alkoholen oder in Mischungen aus den genannten Alkoholen oder deren Azeotropen mit Wasser bei Temperaturen von 60°C bis 200°C gegebenenfalls unter erhöhtem Druck löst, anschließend die Lösung abkühlt und das ausfallende Produkt abfiltriert und gegebenenfalls wäscht.

Der Vorteil dieses Verfahrens liegt darin, daß man einen technisch leicht durchführbaren Prozeß mit gut zu handhabenden Lösungsmitteln hat, der darüber hinaus mit einer hohen Selektivität abläuft.

Das zu reinigende, rohe 2-Mercaptobenzthiazol wird technisch aus Anilin, Schwefelkohlenstoff und Schwefel unter erhöhtem Druck und bei erhöhter Temperatur gemäß US 1 631 871 hergestellt. Dabei wird ein 2-Mercaptobenzthiazol erhalten, das einen Gehalt an 2-Mercaptobenzthiazol von etwa 80 bis 90 % besitzt.

Erfindungsgemäß wird das rohe 2-Mercaptobenzthiazol in höhersiedenden aliphatischen oder cycloaliphatischen Alkoholen oder deren Mischungen oder deren Azeotropen mit Wasser bei Temperaturen von 60 bis 200°C, vorzugsweise bei Temperaturen von 80 bis 165°C, besonders bevorzugt beim Siedepunkt des eingesetzten Alkohols, gelöst.

Als geeignete aliphatische oder cycloaliphatische Alkohole kommen beispielsweise Isopropanol, n-Butanol oder Cyclohexanol oder das Isopropanol/Wasser-Azeotrop in Frage. Die Menge des eingesetzten Alkohole wird dabei so bemessen, daß man bei dem gewählten Temperaturbereich eine gesättigte Lösung des rohen 2-Mercaptobenzthiazols erhält.

Bei dem erfindungsgemäßen Reinigungsverfahren kann zur Lösung des rohen 2-Mercaptobenzthiazols in den genannten Alkoholen unter Druck gearbeitet werden (1 bis 50 bar). Bevorzugt wird jedoch bei atmosphärischem Druck gearbeitet. Das Arbeiten unter Druck kann unter einer Inertgasatmosphäre (Stickstoff, Edelgase) durchgeführt werden.

Die Fällung des in den genannten Alkoholen gelösten 2-Mercaptobenzthiazols kann auf verschiedene Weise durchgeführt werden. Eine Möglichkeit ist die langsame Abkühlung der heißen Lösung auf Temperaturen im Bereich von 0 bis 30°C, bevorzugt auf Raumtemperatur. Der für die Abkühlung benötigte Zeitraum beträgt etwa 30 Minuten bis 10 Stunden, bevorzugt 4 bis 6 Stunden. Das ausgefallene 2-Mercaptobenzthiazol kann dann durch Filtrieren von der Mutterlauge abgetrennt werden.

Eine weitere Möglichkeit der Fällung besteht darin, daß das Lösungsmittel unter vermindertem Druck von etwa 0,01 bar bis 0,9 bar, bevorzugt bei 0,1 bis 0,8 bar, bei konstanter Temperatur teilweise abdestilliert wird. Das aus dieser Lösung ausfallende 2-Mercaptobenzthiazol kann dann entweder in der Hitze oder nach teilweisem oder vollständigem Abkühlen der Lösung abfiltriert werden.

Eine andere Möglichkeit zur Fällung des gelösten 2-Mercaptobenzthiazols ist die Entspannung einer heißen, unter Druck hergestellten Lösung. Das ausgefallene Produkt kann dann sowohl aus der heißen als auch aus der teilweise oder ganz erkalteten Lösung abfiltriert werden. Die Möglichkeiten der Ausfällung des Produkts aus der gesättigten Lösung sind aber nicht auf die hier beschriebenen Varianten beschränkt.

Das abfiltrierte Produkt kann anschließend mit einem Lösungsmittel, bevorzugt mit einem aliphatischen oder cycloaliphatischen Alkohol oder deren Gemischen, gewaschen werden, um restliche anhaftende Mutterlauge zu entfernen. Die Wäsche kann bei Temperaturen von 0 bis 40°C, bevorzugt bei Raumtemperatur, durchgeführt werden. Zur Wäsche werden üblicherweise 0,1 bis 1,0 Gew.-Teile an Lösungsmittel, bezogen auf 1 Gew.-Teil eingesetztes rohes 2-Mercaptobenzthiazol, eingesetzt. Bevorzugt werden 0,3 bis 0,6 Gew.-Teile an Lösungsmittel verwendet.

Die anschließende Trocknung des erhaltenen, gereinigten 2-Mercaptobenzthiazols kann bei einer Temperatur von 20 bis 100°C, bevorzugt bei 30 bis 60°C, gegebenenfalls unter vermindertem Druck durchgeführt werden.

Gemäß einer anderen Verfahrensweise wird das rohe 2-Mercaptobenzthiazol in den vorgenannten bzw. nachstehend aufgeführten Lösungsmitteln nicht gänzlich gelöst sondern nur aufgeschlämmt und ähnlich der zuvor beschriebenen Arbeitsweise aufgearbeitet.

Als Lösungsmittel die zur Aufschlämmung des rohen 2-Mercaptobenzthiazols dienen, können neben den zuvor genannten Lösungsmitteln auch aliphatische oder cycloaliphatische Ether, wie tert.-Butylmethylether, organische Säuren oder deren Ester, wie Essigsäure und Essigsäurealkylester eingesetzt werden.

Dabei werden das Lösungsmittelvolumen, die Zeit und die Temperatur so eingestellt, daß eine optimale Ausbeute bei hoher Reinheit zu erzielen ist. Eine vollständige Lösung der Rohschmelze des 2-Mercaptobenzthiazols ist dabei nicht notwendig um ein reines Produkt zu erhalten.

Bevorzugt werden etwa 0,1 bis 1,0 Gew.-Teile an Lösungsmittel, bezogen auf 1 Gew.-Teil eingesetztes rohes 2-Mercaptobenzthiazol, eingesetzt. Ganz besonders bevorzugt werden 0,2 bis 0,8 Gew.-Teile Lösungsmittel verwendet.

Die Aufschlämmung wird etwa 10 Minuten bis 6 Stunden, bevorzugt 30 Minuten bis 2 Stunden, gerührt. Die Temperatur bei der die Aufschlämmung behandelt wird beträgt etwa 10 bis 100°C, bevorzugt 30 bis 80°C, jedoch liegt sie auf jeden Fall unter der Siedetemperatur des eingesetzten Lösungsmittels oder Lösungsmittelgemisches.

Das gereinigte 2-Mercaptobenzthiazol wird durch Filtration aus der teilweise oder ganz erkalteten Aufschlämmung, bevorzugt bei 20°C, gewonnen.

Das nach dem erfindungsgemäßen Verfahren gereinigte 2-Mercaptobenzthiazol ist von hellgelber Farbe. Die erzielbaren Ausbeuten und Wirkstoffgehalte sind abhängig vom eingesetzten Lösungsmittel und vom Verhältnis von 2-Mercaptobenzthiazol zu eingesetztem Lösungsmittel.

Das nach dein erfindungsgemäßen Verfahren gereinigte 2-Mercaptobenzthiazol kann beispielsweise als Vulkanisationsbeschleuniger oder als Korrosionsschutzmittel verwendet werden.

Beispiel 1

50 g Rohschmelze des 2-Mercaptobenzthiazols (Wirkstoffgehalt: 79,1 %) werden in 140 ml Isopropanol in der Siedehitze unter Stickstoff als Schutzgas gelöst. Anschließend wird das Gemisch zum Abkühlen bei Raumtemperatur stehengelassen. Das ausgefallene Produkt wird abfiltriert, mit 20 ml Isopropanol gewaschen und getrocknet. Man erhält ein hellgelbes Produkt (Ausbeute: 69,8 %, bezogen auf reines 2-Mercaptobenzthiazol; Schmp. 180,1 bis 181,1°C; Wirkstoffgehalt: 98,1 %).

## Beispiel 2

Analog Beispiel 1 werden 50 g rohes 2-Mercaptobenzthiazol (Wirkstoffgehalt: 79,1 %) unter Einsatz von 130 ml eines Gemisches aus 87,4 Gew.-% Isopropanol und 12,6 Gew.-% Wasser (azeotrope Zusammensetzung) unter Stickstoff als Schutzgas umkristallisiert. Das ausgefallene Produkt wird abfiltriert und mit 20 ml des Isopropanol/Wasser-Gemisches gewaschen. Man erhält ein hellgelbes Produkt (Ausbeute: 73,8 %, bezogen auf reines 2-Mercaptobenzthiazol; Schmp. 180,1 bis 181,1°C; Wirkstoffgehalt: 97,0 %).

## Beispiel 3

Analog Beispiel 1 werden 50 g rohes 2-Mercaptobenzthiazol (Wirkstoffgehalt; 89,2 %) unter Einsatz von 39 ml n-Butanol unter Stickstoff als Schutzgas umkristallisiert. Das ausgefallene Produkt wird abfiltriert und mit 20 ml n-Butanol gewaschen. Man erhält ein hellgelbes Produkt (Ausbeute: 84,7 %, bezogen auf reines 2-Mercaptobenzthiazol; Schmp. 179,9 bis 181,2°C; Wirkstoffgehalt: 97,9 %).

## Beispiel 4

Analog Beispiel 1 werden 500 g rohes 2-Mercaptobenzthiazol (Wirkstoffgehalt: 87,7 %) in 100 ml Cyclohexanol unter Stickstoff als Schutzgas gelöst. Das nach Erkalten ausgefallene Produkt wird bei Raumtemperatur abfiltriert und mit 200 ml Cyclohexanol gewaschen. Man erhält ein hellgelbes Produkt (Ausbeute: 87,8 %, bezogen auf reines 2-Mercaptobenzthiazol; Schmp. 180,5 bis 182,0°C; Wirkstoffgehalt: 99,7 %).

## Beispiel 5

50 g rohes 2-Mercaptobenzthiazol (Wirkstoffgehalt: 87,7 %) werden in 10 ml Cyclohexanol in der Siedehitze gelöst und nach Erkalten der Lösung abfiltriert. Der Filterkuchen wird danach mit 50 ml Isopropanol gewaschen. Man erhält ein hellgelbes Produkt (Ausbeute: 86,5 %, bezogen auf reines 2-Mercaptobenzthiazol; Schmp. 180,8 bis 182,0°C; Wirkstoffgehalt: 99,2 %).

## Beispiel 6

Analog Beispiel 5 wird das rohe 2-Mercaptobenzthiazol (Wirkstoffgehalt: 87,7 %) unter Einsatz von 10 ml Cyclohexanol umkristallisiert. Der Filterkuchen wird mit 50 ml eines Gemisches aus 87,4 Gew.-% Isopropanol und 12,6 Gew.-% Wasser gewaschen. Man erhält ein hellgelbes Produkt (Ausbeute: 86,8 %, bezogen auf reines 2-Mercaptobenzthiazol; Schmp. 181,0 bis 182,0°C; Wirkstoffgehalt: 99,1 %).

## Beispiel 7

Analog Beispiel 5 wird das rohe 2-Mercaptobenzthiazol (Wirkstoffgehalt: 87,7 %) unter Einsatz von 10 ml Cyclohexanol umkristallisiert und der Filterkuchen mit 50 ml Methanol gewaschen. Man erhält eine hellgelbes Produkt (Ausbeute: 83,9 %, bezogen auf reines 2-Mercaptobenzthiazol; Schmp. 181,0 bis 182,8°C; Wirkstoffgehalt: 99,3 %).

## Beispiel 8

50 g rohes 2-Mercaptobenzthiazol (Wirkstoffgehalt; 88,3 %) werden in 10 ml Cyclohexanol in der Siedehitze gelöst und das ausgefallene Produkt nach Abkühlen der Lösung auf Raumtemperatur abfiltriert und unmittelbar getrocknet. Man erhält ein hellgelbes Produkt (Ausbeute: 86,7 %, bezogen auf reines 2-Mercaptobenzthiazol; Schmp. 179,1 bis 181,1°C; Wirkstoffgehalt: 97,2 %).

## Beispiel 9

Analog Beispiel 8 werden 50 g rohes 2-Mercaptobenzthiazol (Wirkstoffgehalt: 88,3 %) unter Einsatz von 50 ml n-Butanol umkristallisiert. Das ausgefallene Produkt wird abfiltriert und unmittelbar getrocknet. Man erhält ein hellgelbes Produkt (Ausbeute: 84,0 %, bezogen auf reines 2-Mercaptobenzthiazol; Schmp. 179,1 bis 181,0°C; Wirkstoffgehalt: 95,8 %).

## Beispiel 10

50 g Rohschmelze des 2-Mercaptobenzthiazols (Wirkstoffgehalt: 91,0 %) werden in 50 ml Isopropanol für 30 Minuten bei 30°C gerührt. Nach dem Abkühlen auf Raumtemperatur wird der Rückstand abfiltriert und getrocknet. Man erhält ein hellgelbes Produkt (Ausbeute: 92,1 % bezogen auf reines 2-Mercaptobenzthiazol; Schmp.: 181,8 bis 182,1°C; Wirkstoffgehalt: 99,6 %).

## Beispiel 11

Analog Beispiel 10 wird das rohe 2-Mercaptobenzthiazol (Wirkstoffgehalt: 91,0 %) 1 Stunde in 50 ml Isopropanol aufgeschlämmt. Man erhält ein hellgelbes Produkt (Ausbeute: 91,6 % bezogen auf reines 2-Mercaptobenzthiazol; Schmp.: 181,8 bis 182,1°C; Wirkstoffgehalt: 99,4 %).

## Beispiel 12

Analog Beispiel 10 wird das rohe 2-Mercaptobenzthiazol (Wirkstoffgehalt: 91,0 %) 1,5 Stunden in 50 ml Isopropanol aufgeschlämmt. Man erhält ein hellgelbes Produkt (Ausbeute: 91,6 % bezogen auf reines 2-Mercaptobenzthiazol; Schmp.: 181,2 bis 182,0 ° C; Wirkstoffgehalt: 99,3 %).

Beispiel 13

Analog Beispiel 10 wird das rohe 2-Mercaptobenzthiazol (Wirkstoffgehalt: 91,0 %) 2 Stunden in 80 ml Isopropanol aufgeschlämmt. Man erhält ein hellgelbes Produkt (Ausbeute: 89,5 % bezogen auf reines 2-Mercaptobenzthiazol; Schmp.: 181,2 bis 182,0 ° C; Wirkstoffgehalt: 99,4 %).

Beispiel 14

Analog Beispiel 10 wird das rohe 2-Mercaptobenzthiazol (Wirkstoffgehalt: 91,0 %) 3 Stunden in 50 ml Isopropanol aufgeschlämmt. Man erhält ein hellgelbes Produkt (Ausbeute: 91,0 % bezogen auf reines 2-Mercaptobenzthiazol; Schmp.: 181,2 bis 182,0 ° C; Wirkstoffgehalt: 100,0 %).

Beispiel 15

Analog Beispiel 10 wird das rohe 2-Mercaptobenzthiazol (Wirkstoffgehalt: 87,7 %) 2 Stunden in einem Gemisch aus 87,4 Gew.-% Isopropanol und 12,6 Gew.-% Wasser (azeotrope Zusammensetzung) aufgeschlämmt. Man erhält ein hellgelbes Produkt (Ausbeute: 90,3 % bezogen auf reines 2-Mercaptobenzthiazol; Schmp.: 180,1 bis 181,9 ° C; Wirkstoffgehalt: 98,8 %).

Beispiel 16

Analog Beispiel 10 wird das rohe 2-Mercaptobenzthiazol (Wirkstoffgehalt: 87,7 %) 2 Stunden in 80 ml n-Butanol aufgeschlämmt. Man erhält ein hellgelbes Produkt (Ausbeute: 82,4 % bezogen auf reines 2-Mercaptobenzthiazol; Schmp.: 181,1 bis 182,1 ° C; Wirkstoffgehalt: 99,7 %).

Beispiel 17

Analog Beispiel 10 wird das rohe 2-Mercaptobenzthiazol (Wirkstoffgehalt: 87,7 %) 2 Stunden in 50 ml Cyclohexanol aufgeschlämmt. Man erhält ein hellgelbes Produkt (Ausbeute: 85,5 % bezogen auf reines 2-Mercaptobenzthiazol; Schmp.: 181,0 bis 182,0 ° C; Wirkstoffgehalt: 98,8 %).

Beispiel 18

Analog Beispiel 10 wird das rohe 2-Mercaptobenzthiazol (Wirkstoffgehalt: 79,1 %) 2 Stunden in 50 ml tert.-Butylmethylether aufgeschlämmt. Man erhält ein hellgelbes Produkt (Ausbeute: 90,5 % bezogen auf reines 2-Mercaptobenzthiazol; Schmp.: 180,5 bis 182,0 ° C; Wirkstoffgehalt: 99,3 %).

Beispiel 19

Analog Beispiel 10 wird das rohe 2-Mercaptobenzthiazol (Wirkstoffgehalt: 79,1 %) 2 Stunden in 50 ml Essigsäureethylester (Ethylacetat) aufgeschlämmt. Man erhält ein hellgelbes Produkt (Ausbeute: 87,5 % bezogen auf reines 2-Mercaptobenzthiazol; Schmp.: 181,0 bis 182,0 ° C; Wirkstoffgehalt: 99,4 %).

Beispiel 20

Analog Beispiel 10 wird das rohe 2-Mercaptobenzthiazol (Wirkstoffgehalt: 79,1 %) 2 Stunden in 50 ml konzentrierter Essigsäure aufgeschlämmt. Man erhält ein hellgelbes Produkt (Ausbeute: 93,6 % bezogen auf reines 2-Mercaptobenzthiazol; Schmp.: 180,9 bis 181,9 ° C; Wirkstoffgehalt: 98,7 %).

**Patentansprüche**

1. Verfahren zur Reinigung von rohem 2-Mercaptobenzthiazol, dadurch gekennzeichnet, daß man das rohe 2-Mercaptobenzthiazol in höher-siedenden aliphatischen oder cycloaliphati-schen Alkoholen oder in Mischungen aus den genannten Alkoholen oder deren Azeotropen mit Wasser bei Temperaturen von 60 ° C bis 200 ° C gegebenenfalls unter erhöhtem Druck löst, anschließend die Lösung abkühlt und das ausfallende Produkt abfiltriert und gegebenenfalls wäscht.

2. Verfahren nach Anspruch 1, dadurch gekennzeichnet, daß man aus der erhaltenen alkoholischen Lösung des rohen 2-Mercaptobenzthiazols den Alkohol bei Temperaturen von 60 bis 200 ° C gegebenenfalls unter erniedrigtem Druck verdampft und das dabei ausfallende Produkt abfiltriert und gegebenenfalls wäscht.

3. Verfahren nach Anspruch 1, dadurch gekennzeichnet, daß man die alkoholische Lösung des 2-Mercaptobenzthiazols, die bei erhöhtem Druck hergestellt wurde, entspannt, gegebenenfalls die Temperatur langsam auf Raumtemperatur senkt und das dabei ausfallende Produkt abfiltriert und gegebenenfalls wäscht.

4. Verfahren zur Reinigung von rohem 2-Mercaptobenzthiazol, dadurch gekennzeichnet, daß

man das rohe 2-Mercaptobenzthiazol in höhersiedenden aliphatischen oder cycloaliphatischen Alkoholen, aliphatischen oder cycloaliphatischen Ethern, organischen Säuren oder deren Estern bei Temperaturen von 10 bis 100 °C aufschlämmt, anschließend die Lösung abkühlt und das ausfallende Produkt abfiltriert und gegebenenfalls wäscht.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A-2 617 479 (SOCIETE MANUFACTURE LANDAISE DE PRODUITS CHIMIQUES) <br> * Ansprüche * <br><br> ----- | 1 | C07D277/72 |
| | | | **RECHERCHIERTE SACHGEBIETE (Int. Cl.5)** <br><br> C07D |

Der vorliegende Recherchenbericht wurde für alle Patentansprüche erstellt

| Recherchenort | Abschlußdatum der Recherche | Prüfer |
|---|---|---|
| DEN HAAG | 25 AUGUST 1992 | HENRY J.C. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.............................................................................
& : Mitglied der gleichen Patentfamilie, übereinstimmendes
    Dokument

EPO FORM 1503 03.82 (P0403)